Europäisches Patentamt

European Patent Office

Office européen des brevets

(11) Publication number: **0 127 014**
A2

# EUROPEAN PATENT APPLICATION

(21) Application number: 84105047.9

(22) Date of filing: 04.05.84

(51) Int. Cl.³: **B 25 G 1/10**

(30) Priority: 31.05.83 US 499542

(43) Date of publication of application:
05.12.84 Bulletin 84/49

(84) Designated Contracting States:
AT BE CH DE FR GB IT LI LU NL SE

(71) Applicant: Smith, Lloyd T.
715 West Broadway
Newton Kansas 67114(US)

(72) Inventor: Smith, Lloyd T.
715 West Broadway
Newton Kansas 67114(US)

(72) Inventor: Berry, Loren E.
1401 Westborough
Newton Kansas 67114(US)

(72) Inventor: Ediger, Glen W.
127 South Main
Newton Kansas 67114(US)

(72) Inventor: Hindman, Thomas E.
127 N. Bleckley
Wichita Kansas 67208(US)

(72) Inventor: Ten Eyck, Richard E.
7456 South Seneca
Wichita Kansas 67208(US)

(74) Representative: UEXKÜLL & STOLBERG Patentanwälte
Beselerstrasse 4
D-2000 Hamburg 52(DE)

(54) Hand grip for hand tools.

(57) A hand grip for hand tools has a generally bulbar configuration conforming to the shape of the palm of the hand when clenched in grasping relation to the hand grip. A shank extending from the hand grip adjacent its normally uppermost end has opposed grooves for receiving two fingers which grip the shank The longitudinal axis of the shank is parallel with an extension of the axis of rotation of the forearm and the wrist while the latter is straight and unbent.

Fig. 1

EP 0 127 014 A2

## HAND GRIP FOR HAND TOOLS

It has been recognized that a multiplicity of diseases, attributable to improperly designed hand tools, may soon reach epidemic proportions. The motions of the hand, wrist and forearm during use of such tools are irregular and oftentimes somewhat intense, causing permanent injury in many instances. The problem becomes acute when the tools are used continuously over long periods of time, requiring all too frequently the need for rest or physical therapy and even remedial surgery, with resultant loss of workmen time if not substantial incapacity.

Certain of the solutions heretofore suggested include bent shanks, curved or bent handles, handles wrapped with soft plastics, yieldable grip portions, designs intended to prevent or reduce wrist bending and configurations which permit the shanks to straddle the thumb and forefinger. But no such suggestion has completely or satisfactorily solved the problem.

Keeping in mind, therefore, the insufficiencies and other limitations of the hand, its fingers, the wrist and the forearm, as well as the nature of the tasks to be performed, we have provided a hand grip for use with a wide variety of tools which has, as an important attribute, the avoidance of stress concentrations effected primarily by equal distribution of the compression forces throughout the entire surface area of the palm of the hand.

As distinguished from pistol grip and spherical configurations which undesirably frequent the marketplace, and distinguishable from a mere elongated, relatively small diameter grip portion, we employ a smooth, bulbar configuration which fills and conforms to the shape of the palm. The accompanying shank is disposed to be straddled between and gripped by the forefinger and the next adjacent middle finger, and the axis of the shank is properly related to the axis of rotation of the forearm and unbent wrist.

The hand grip portion is of such nature as to permit, if desired, top engagement by the ball of the thumb and a partial wrap-around below the shank by three fingers to force the hand grip portion into a tight, non-slip snug fit into the palm. The user is, therefore, able to maintain complete control not only in regard to imparted thrust but to impede rotation of the shank about its longitudinal axis. Thus, inasmuch as the wrist is kept straight and all motion patterns have been taken into consideration, the likelihood of injuries has been reduced if not entirely eliminated.

In the drawings:

Figure 1 is a side elevational view of a hand grip for hand tools made in accordance with our present invention showing the same in association with a user's arm and grasped by the hand;

Fig. 2 is an enlarged view showing in elevation the side thereof depicted in Fig. 1;

Fig. 3 is an elevational view showing the rear end thereof;

Fig. 4 is an elevational view showing the front end thereof;

Fig. 5 is a top plan view thereof;

Fig. 6 is a rear elevational view thereof; and

Fig. 7 is a view showing the bottom of the hand grip.

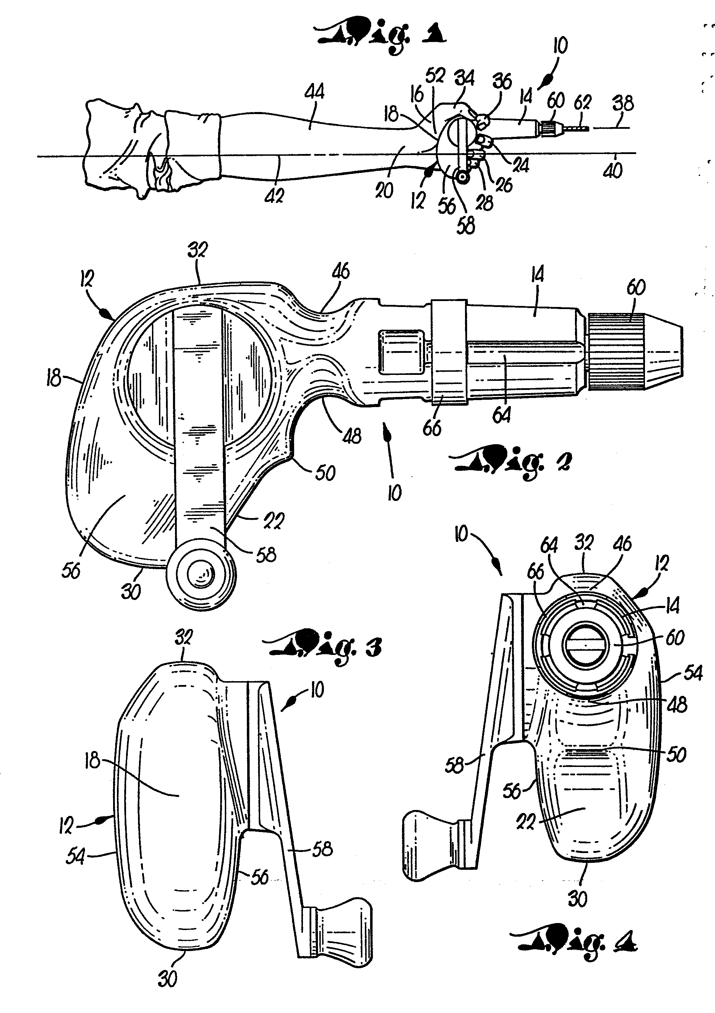

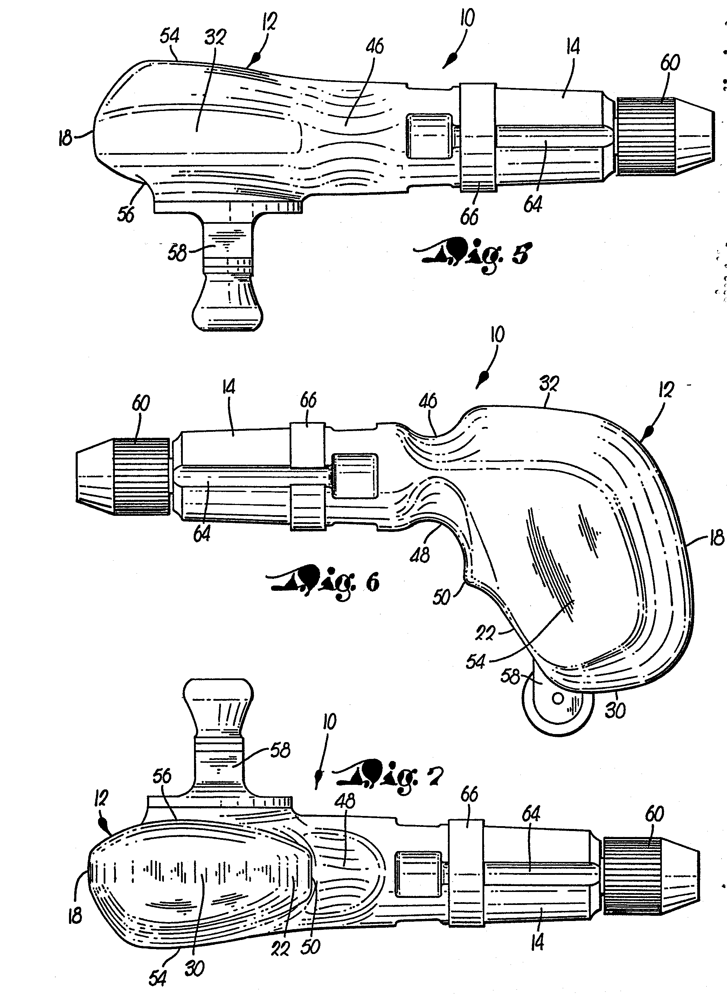

The grip 12 is shaped and sized to substantially fill, snugly fit and smoothly engage the palm of the hand 16 as shown in Fig. 1. The grip 12 essentially conforms to the concave, cupped configuration of the hand 14 when clenched as illustrated.

To these ends, the grip 12, when so grasped, has a normally rearmost, elongated, longitudinally and transversely convex edge 18 adapted to receive a straight thrust imparted thereto by the hand 16 adjacent the wrist 20. The grip 12 is also provided, when grasped by the hand 16, a normally forwardmost, elongated, longitudinally and transversely convex edge 22. In use, the edge 22 is wrapped by the middle fingers 24 and 26 and the little finger 28 of the hand 16.

Additionally, the grip 12, once again, when grasped by the hand 16, is provided with a normally lowermost, elongated, longitudinally and transversely convex free end 30 adjacent the finger 28. Further, there is provided an elongated, longitudinally and transversely convex end 32 which is normally uppermost when grasped by the hand 16. The end 32 is disposed for engagement, when so desired, by the ball of the thumb 34 of the hand 16.

The shank 14, at the end 32, extends forwardly from the edge 22 and is disposed for a straddling grasp at the grip 12 between the forefinger 36 and the finger 24 of the hand 16 for impeding rotation of the shank 14 about its longitudinal axis 38. Noteworthy is the fact that the axis 38 is disposed in parallelism with an extension 40 of the axis 42 of rotation of the forearm 44 and of the straight, unbent wrist 20 when the grip 12 is grasped.

The shank 14 has a transverse, top groove 46 adjacent the grip 12 for receiving the finger 36 and a transverse, bottom groove 48 adjacent the grip 12 for receiving the finger 24. The concave grooves 46 and 48 are, in effect, portions of the end 32 and edge 22 respectively and are smoothly uninterrupted as they cooperate to essentially merge together as a substantial wrap around of the shank 14. The groove 46 opens upwardly whereas the groove 48 opens downwardly and forwardly, defined by a rounded, lowermost terminus 50 disposed transversely of the edge 22.

Accordingly, th edges 18 and 22 and the ends 30 and 32 present a smooth, continuous, uninterrupted arch extending from the groove 46 to the terminus 50 of the groove 48 such that the edge 18 fits snugly at the end 32 against the base 52 of the palm of the hand 16 adjacent the thumb 34. Thus, the compressive forces of the hand 16 on the grip 12 are evenly distributed throughout the surface area of the palm when the grip 12 is grasped whereby to obtain both maximum leverage and control.

To this end also, the grip 12 has a pair of sides 54 and 56 which are convex longitudinally (between ends 30 and 32) and transversely (between edges 18 and 22) with the width of the grip 12 (between sides 54 and 56) progressively increasing as the edge 18 is approached. The sides 54 and 56 merge in a roundish smoothness with the ends 30 and 32, the edges 18 and 22 and the notches 46 and 48, and fade continuously and evenly into the shank 14.

Forming no part of the present invention is a crank 58 on the side 56 which, when manually turned, rotates a chuck 60 (on the shank 14) and a drill bit 62 carried by the chuck 60 through mechanism (not shown) in the grip 12 and the shank 14; bit storage grooves 64 in the shank 14 are releasably held in place by a C-band 66 rotatable on the shank 14.

In use, as demonstrated in Fig. 1, the nature of the grip 12, as shown and above described, somewhat automatically causes the operator to grasp the tool 10 and place it in use without bending the wrist 20 such that counter-rotative forces imparted by the bit 62 to the shank 14 and the grip 12 are easily resisted. Quite naturally also is the thrust to be imparted to the bit 62 from the base 52 of the palm of the hand 16, all without tiredness or likelihood of injury to the hand 16, wrist 20 or forearm 44 even after long, continuous periods of use day after day.

There is virtually no risk of the grip 18 slipping from within or turning in the palm of the hand 16, eliminating skin blisters and calluses, muscular soreness and other conditions characterized by inflammation or pain in bones, ligaments, joints and fibrous tissue. All such safety measures result from perfection in the fit of the grip 12 within the hand 16 and the naturalness of the user keeping his wrist 20 unbent and maintaining the axis 38 parallel with the extension 40 of the axis 42.

The grip 12 and its shank 14 are adapted, with the same features and advantages, for many uses other than as a part of a drill. In those cases, especially when the crank 58 is omitted, the user might desire to place the ball of his thumb 34 in engagement with the side 56 adjacent the end 32. By way of example only, with or without the chuck 60 or its equivalent, attachments to the shank 14 include such hand tools as brooms, cutters, sanders, planes, push drills, scrapers, screwdrivers and wrenches. In each instance, torque and/or thrust can be easily controlled by the operator.

- 1 -

0127014

Claims.

1. In a hand tool, an elongated, generally bulbar, hand grip portion shaped and sized to substantially fill, snugly fit and smoothly engage the palm of the hand and essentially conform to the concave, cupped configuration of the clenched hand, whereby the compressive forces of the hand on the grip portion are evenly distributed throughout the surface area of the palm when said portion is grasped to obtain both maximum leverage and control.

2. The invention of Claim 1, said portion having, when grasped by the hand, a normally rearmost, elongated, longitudinally and transversely convex edge adapted to receive a straight thrust imparted thereto by the hand adjacent the wrist.

3. The invention of Claim 1, said portion having, when grasped by the hand, a normally forwardmost, elongated, longitudinally and transversely convex edge wrapped by the middle and little fingers of the hand.

4. The invention of Claim 1, said portion having, when grasped by the hand, a normally lowermost, elongated, longitudinally and transversely convex free end adjacent the little finger of the hand.

5. The invention of Claim 1, said portion having, when grasped by the hand, a normally uppermost, elongated, longitudinally and transversely convex end disposed for engagement, when so desired, by the ball of the thumb of the hand.

6. The invention of Claim 5, said portion having, when grasped by the hand, a normally forwardmost, elongated, longitudinally and transversely convex edge looped by the middle and little fingers of the hand for maintaining the grip portion tightly engaged in said palm.

7. The invention of Claim 6, said grip portion having, when grasped by the hand, an elongated shank portion at said uppermost end extending forwardly from said forwardmost edge and disposed for a straddling grasp at said grip portion between the forefinger and next adjacent middle finger of the hand for impeding rotation of the shank portion about its longitudinal axis.

8. The invention of Claim 7, said axis of the shank portion being disposed in parallelism with an extension of the axis of rotation of the forearm and straight, unbent wrist when the grip portion is grasped.

9. The invention of Claim 7, said shank having a transverse, top groove adjacent the grip portion for receiving said forefinger.

10. The invention of Claim 7, said shank having a transverse, bottom groove adjacent the grip portion for receiving said next adjacent middle finger.

11. The invention of Claim 10, said shank having a transverse, top groove adjacent the grip portion for receiving said forefinger.

12. The invention of Claim 11, said portion having, when grasped by the hand, a normally rearmost, elongated, longitudinally and transversely convex edge adapted to receive a straight thrust imparted thereto by the hand adjacent the wrist.

13. The invention of Claim 12, said portion having, when grasped by the hand, a normally forwardmost, elongated, longitudinally and transversely convex edge wrapped by the middle and little fingers of the hand.

14. The invention of Claim 13, said portion having, when grasped by the hand, a normally lowermost, elongated, longitudinally and transversely convex free end adjacent the little finger of the hand.

15. The invention of Claim 14, said edges and said ends presenting a continuous, uninterrupted arch extending from said top groove to said bottom groove.

*Fig. 1*

*Fig. 2*

*Fig. 3*

*Fig. 4*

Fig. 5

Fig. 6

Fig. 7